# EUROPÄISCHE PATENTSCHRIFT

(11) **EP 2 120 767 B1**
(45) Veröffentlichungstag und Bekanntmachung des Hinweises auf die Patenterteilung: **21.07.2010**
(21) Anmeldenummer: 08716102.2
(22) Anmeldetag: 28.02.2008
(51) Int. Cl.: A61B 19/00

(54) **VORRICHTUNG ZUM DEHNEN DER HAUT**
DEVICE FOR STRETCHING THE SKIN
DISPOSITIF POUR ÉTIRER LA PEAU

(30) Priorität: 08.03.2007 DE 102007011570
(43) Veröffentlichungstag der Anmeldung: 25.11.2009
(73) Patentinhaber: Fleischmann, Wilhelm, Dr. med., 74321 Bietigheim-Bissingen (DE)
(72) Erfinder: Fleischmann, Wilhelm, Dr. med., 74321 Bietigheim-Bissingen (DE)
(74) Vertreter: Patentanwälte Westphal, Mussgnug & Partner
(86) Internationale Anmeldenummer: PCT/EP2008/001574
(87) Internationale Veröffentlichungsnummer: WO 2008/107110

(56) Entgegenhaltungen:
- FR-A- 2 756 722
- US-A- 4 896 680

## Beschreibung

Die Erfindung betrifft eine Vorrichtung zum Dehnen der Haut.

Das Dehnen der Haut (Skin-Stretching) wird in der Chirurgie angewendet, um Haut für eine Transplantation zu gewinnen und insbesondere um großflächige Wunden durch Zusammenziehen der Wundränder zu schließen.

Aus der DE 44 44 130 A1 ist es bekannt, auf in der Richtung der erwünschten Dehnung der Haut verlaufenden Führungsstangen zwei Verankerungselemente mittels einer Gewindespindel gegeneinander verstellbar zu bewegen. Die Verankerungselemente sind durch Backen gebildet, die in die Haut einstechbare Haken tragen. Die Haken der beiden Verankerungselemente werden in die einander gegenüberliegenden Ränder einer Wunde eingestochen, so dass sich die Wunde in dem Zwischenbereich zwischen den Verankerungselementen befindet. Werden die Verankerungselemente gegeneinander bewegt, so werden die Wundränder zusammengezogen. Die dabei auf die Haut der Wundränder wirkende Zugkraft führt zu einer verstärkten Gewebeproliferation, die das Zusammenziehen der Wundränder begünstigt und ermöglicht.

Aus der US 4 896 680 ist eine Vorrichtung bekannt, bei welcher als Führungseinrichtung ein quer geripptes flexibles Band dient. Das eine Ende des Bandes wird mittels eines sicherheitsnadel-förmigen Verankerungselementes in der Haut fixiert. Ein zweites solches Verankerungselement ist auf dem Band verschiebbar geführt und verrastet in den Querrippen des Bandes.

Aus der US 5 571 138 ist es bekannt, zur Gewinnung von Haut für eine Transplantation ein flexibles Band als Führungseinrichtung zu verwenden, auf welchem zwei Verankerungselemente verschiebbar und klemmbar angeordnet sind, um die Haut beiderseits des Zwischenbereichs zwischen diesen Verankerungselementen zu dehnen und in dem Zwischenbereich überschüssige Haut für eine Transplantation zu gewinnen.

Aus der US 5 531 790 ist es bekannt, zur Behandlung der Kopfkahlheit die seitlichen behaarten Hautpartien des Kopfes durch eine Vorrichtung zu dehnen und zusammenzuziehen, die aus einem elastischen Band besteht, welches an seinen beiden Enden eine oder mehrere Reihen von Haken als Verankerungselemente trägt. Die Haken werden in die behaarte Kopfhaut eingestochen, so dass diese durch die Elastizität des Bandes zusammengezogen wird. Durch die Anordnung mehrerer Reihen von Verankerungselementen kann ein in Dehnungsrichtung größerer Hautbereich erfasst und gedehnt werden. Eine Verstellbarkeit der Verankerungselemente ist nicht vorgesehen.

Der Erfindung liegt die Aufgabe zugrunde, eine Vorrichtung zum Dehnen der Haut zu schaffen, die eine verbesserte Effektivität der Hautproliferation ermöglicht.

Diese Aufgabe wird erfindungsgemäß gelöst durch eine Vorrichtung mit den Merkmalen des Patentanspruchs 1.

Vorteilhafte Ausführungen der Erfindung sind in den Unteransprüchen angegeben.

Bei der erfindungsgemäßen Vorrichtung wird ein Paar von ersten Verankerungselementen in der Haut verankert. Diese ersten Verankerungselemente werden verstellbar gegeneinander geführt, wodurch der Abstand zwischen den ersten Verankerungselementen und der Zwischenbereich zwischen diesen Verankerungselementen verkleinert wird. Die Haut wird beiderseits außerhalb der ersten Verankerungselemente gedehnt. Bei der häufigsten Anwendung für einen Wundverschluss werden die Wundränder zusammengezogen, um die zwischen den Verankerungselementen liegende Wundfläche zu verkleinern und die an die Wundränder anschließenden Hautareale zu dehnen.

Die Zugkraft, die durch diese ersten Verankerungselemente auf die Haut ausgeübt werden kann, ist begrenzt. Wird die Zugkraft zu groß, können die Verankerungselemente aus der Haut ausreißen. Auch wenn die Verankerungselemente nicht ausreißen, kann auf das in Zugrichtung vor den Verankerungselementen liegende Hautgewebe ein so hoher Druck ausgeübt werden, dass das Gewebe ischämisch und bei längerer Aufrechterhaltung der Zugkraft dadurch nekrotisch wird.

Diesem Nachteil wirkt die Erfindung dadurch entgegen, dass in Zugrichtung hinter wenigstens einem der ersten Verankerungselemente wenigstens ein zweites Verankerungselement angeordnet ist. Dieses zweite Verankerungselement wird ebenfalls in der Haut verankert und weist aufgrund seiner Anordnung einen größeren Abstand von dem Zwischenbereich zwischen den ersten Verankerungselementen, z. B. von dem Wundrand, auf. Das wenigstens eine zweite Verankerungselement ist ebenfalls auf der Führungseinrichtung verstellbar. Die zweiten Verankerungselemente können durch ihre Verstellbarkeit eine einstellbare Zugkraft auf die Haut ausüben, die in einem größeren Abstand von dem Wundrand wirksam wird. Dadurch ergeben sich folgende wesentliche Vorteile für die Hautdehnung.

Experimentelle Untersuchungen haben gezeigt, dass die auf die Haut einwirkende Zugkraft nur eine geringe Reichweite hat. Durch die Nachgiebigkeit der Haut wird die Zugkraft über eine Strecke von wenigen Zentimetern abgebaut, sodass in einer Entfernung von bei der menschlichen Haut im allgemeinen etwa 5 bis 6 cm von der Einleitung der Zugkraft diese Zugkraft keine Wirkung mehr entfaltet und keine Gewebeproliferation stimuliert. Durch eine oder mehrere zweite Verankerungselemente wird jeweils in einem entsprechend größeren Abstand von dem Wundrand und dem ersten Verankerungselement eine zusätzliche Zugkraft auf die Haut ausgeübt, sodass ein wesentlich größeres Hautareal für die Dehnung und die Gewebeproliferation genutzt werden kann.

Durch die zweiten Verankerungselemente wird die Haut bereits vor dem ersten Verankerungselement unter eine Zugkraft gesetzt, sodass die durch die zweiten Verankerungselemente vorbelastete Haut durch das in Zugrichtung letzte erste Verankerungselement nur noch mit einer geringeren relativen Zugkraft beaufschlagt werden muss. Durch die einzelnen Verankerungselemente und insbesondere durch die ersten Verankerungselemente wirkt daher jeweils nur eine geringere Kraft an den Verankerungspunkten auf der Haut ein. Dadurch wird ein Ausreißen der ersten Verankerungselemente vermieden. Auch der durch die ersten Verankerungselemente auf das Hautgewebe vor diesen Verankerungselementen ausgeübte Druck kann unter die Ischämie-Grenze reduziert werden, ohne dass dadurch die Gewebeproliferation verschlechtert wird. Die Hautdehnung kann daher auch über einen langen Zeitraum ohne Gewebeschädigung durchgeführt werden, was eine schnellere und effektivere Behandlung zur Folge hat.

Die durch die Verankerungselemente in die Haut eingeleitete Zugkraft kann so groß gewählt werden, dass auf der Druckseite der Verankerungselemente eine Ischämie entsteht. Vor Erreichen der Ischämietoleranz des Gewebes, d. h. spätestens nach einigen Stunden muss die Zugkraft aufgehoben oder zumindest soweit reduziert werden, dass auf der Druckseite der Verankerungselement eine Wiederdurchblutung des Gewebes möglich ist. Bei einer solchen zyklischen Dehnung, liegen zwischen den Zeitintervallen der Dehnung und damit der Stimulation der Gewebeproliferation jeweils zeitliche Regenerationsintervalle, in welchen die Haut nicht gedehnt wird.

Da die ersten und zweiten Verankerungselemente voneinander unabhängig auf der Führungseinrichtung verstellt werden können, können auch die durch die ersten und zweiten Verankerungselemente auf die Haut einwirkenden Zugkräfte unabhängig voneinander variiert werden. Es können bspw. einzelne der Verankerungselemente auf eine hohe Zugkraft eingestellt werden, die eine starke Proliferation bewirkt, wobei auf der Druckseite der Verankerungselemente ggf. auch die Ischämie-Grenze überschritten werden kann. Bei den anderen Verankerungselementen wird gleichzeitig die Zugkraft so reduziert, dass sich das Hautgewebe vor diesen Verankerungselementen wieder bei guter Blutperfusion erholen kann. In entsprechenden Zeitzyklen wird die Funktion der Verankerungselemente vertauscht, sodass sich nun das Hautgewebe bei den zuvor unter hoher Zugspannung stehenden Verankerungselementen erholen kann und die Zugkraft über die Verankerungselemente ausgeübt wird, die zuvor entlastet waren. Da hierbei stets ein Teil der Verankerungselemente eine hohe Zugkraft auf die Haut ausübt, steht die Haut ständig unter einer für die Gewebeproliferation vorteilhaften hohen Zugkraft, ohne dass eine Gewebeschädigung auftritt. Die Einleitung dieser hohen Zugkraft in die Haut wechselt dabei zyklisch ihre Position.

Bei der Wundbehandlung können auch Situationen auftreten, bei welchen die Haut nur im Bereich eines Wundrandes gedehnt werden soll, während eine Dehnung der Haut im gegenüberliegenden Wundrand unerwünscht ist, z. B. weil sich unter diesem Wundrand eine Knochenkante befindet. In einer solchen Situation kann die erfindungsgemäße Vorrichtung vorteilhaft in der Weise eingesetzt werden, dass an dem Wundrand, an welchem keine Hautdehnung stattfinden soll, das erste Verankerungselement und nachfolgende zweite Verankerungselemente so in der Haut verankert und auf der Führungseinrichtung eingestellt werden, dass die zweiten Verankerungselemente keine Zugkraft auf die Haut ausüben. Die zweiten Verankerungselemente dienen hierbei nur dazu, die Vorrichtung auf der einen Seite der Wunde in einem großflächigen Hautareal ohne Hautdehnung zu fixieren, um die auf den anderen Wundrand für die Hautdehnung einwirkende Zugkraft abzustützen.

Die Führungseinrichtung, die Verankerungselemente und die Verstellung der Verankerungselemente auf der Führungseinrichtung können in unterschiedlicher Weise ausgebildet sein. Wesentlich ist, dass die Verankerungselemente auf der Führungseinrichtung verstellbar sind und in ihrer jeweiligen Verstellposition gehalten werden, um die jeweilige Zugkraft des einzelnen Verankerungselementes individuell einstellen und aufrechterhalten zu können. Die Führungseinrichtung kann aus starren Führungen oder einem flexiblen Band bestehen. Die Verankerungselemente können in die Haut einstechbare Haken sein, die einzeln oder in Reihen in den Verankerungselementen angeordnet sind. Ebenso können die Verankerungselemente sicherheitsnadel-förmig in der Haut verankert werden. Die Zugkraft, die durch die Verankerungselemente auf die Haut ausgeübt wird, kann durch die Positionsverstellung der Verankerungselemente und damit durch die Dehnung der Haut gegen deren Elastizität bewirkt werden. Zusätzlich können noch Federkräfte die Zugkraft verstärken und beeinflussen.

Die Verstellung der Verankerungselemente kann in einfacher Weise manuell erfolgen, z. B. entsprechend einem vorgegebenen Zeitplan oder entsprechend einer jeweils aktuellen Beurteilung der Haut- und Wundsituation. Ebenso ist es möglich, die Verstellung der Verankerungselemente über motorische Antriebssysteme zu bewirken. Solche Antriebssysteme können dabei nach einem vorgegebenen oder programmierbaren Plan zeitlich und/oder in Bezug auf die jeweilige Zugkraft gesteuert werden. Schließlich ist auch eine geregelte automatische Verstellung der Verankerungselemente möglich. Hierzu wird vorzugsweise die jeweils von den Verankerungselementen in die Haut eingeleitete Zugkraft gemessen, die von der aktuell erfolgten Hautdehnung abhängig ist, und die Zugkraft der Verankerungselemente wird auf einen vorgebbaren Sollwert nachgeregelt. Damit ist eine Optimierung der Hautdistraktion über längere Zeiträume ohne Personalaufwand möglich.

Im Folgenden wird die Erfindung anhand von in der Zeichnung dargestellten Ausführungsbeispielen näher erläutert. Es zeigen
- Fig. 1: eine Seitenansicht einer Vorrichtung zum Dehnen der Haut in einer ersten Ausführung,
- Fig. 2: eine Ansicht der Vorrichtung der Fig. 1 von unten,
- Fig. 3: eine Ansicht von unten einer Abwandlung dieser Vor- richtung,
- Fig. 4: eine Ansicht von unten einer weiteren Abwandlung die- ser Vorrichtung,
- Fig. 5: ein Detail der Vorrichtung der ersten Ausführung in einem Vertikalschnitt,
- Fig. 6: eine Ansicht des Details der Fig. 5 von oben,
- Fig. 7: eine Ansicht einer zweiten Ausführung der Vorrichtung von oben und
- Fig. 8: ein Anwendungsbeispiel der Vorrichtung.

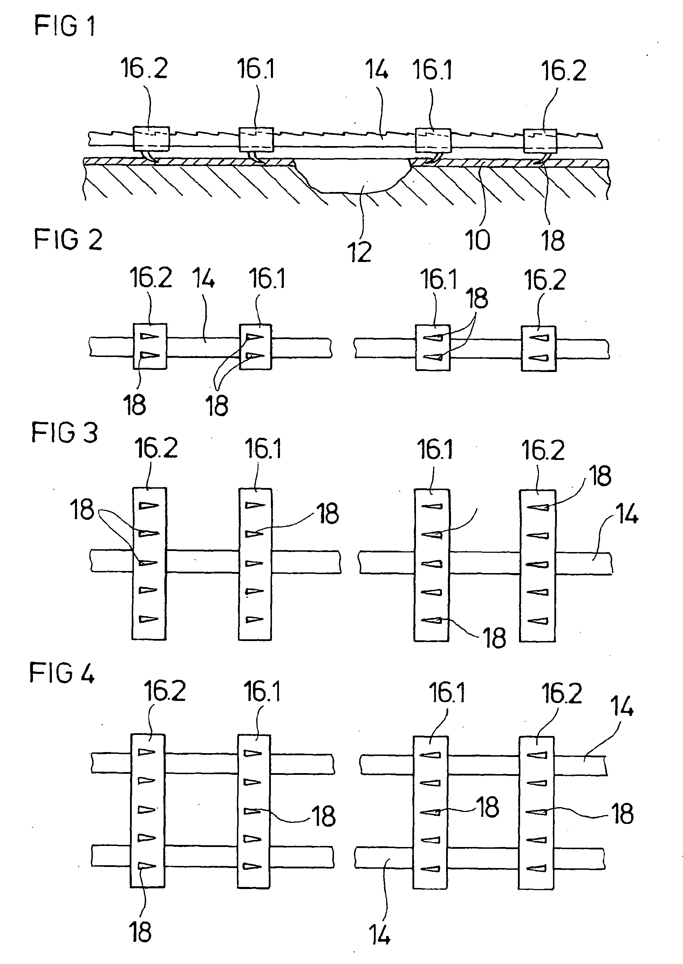

In dem ersten Ausführungsbeispiel der Figuren 1 und 2 ist eine Vorrichtung zum Dehnen der Haut gezeigt. In Fig. 1 ist eine der wichtigsten Anwendungen dieser Vorrichtung dargestellt, nämlich das Dehnen der Haut 10 an den Rändern einer Wunde 12, um die Haut 10 der Wundränder zusammenzuziehen und die Wunde 12 zu verschließen.

Die Vorrichtung weist eine Führungseinrichtung auf, die in dem Ausführungsbeispiel der Figuren 1 und 2 als flexibles Band 14 ausgebildet ist, welches vorzugsweise aus Kunststoff hergestellt ist. Auf dem Band 14 sind Verankerungselemente 16 angeordnet. Die Verankerungselemente 16 sind auf dem Band 14 in Längsrichtung des Bandes 14 verschiebbar und in ihrer jeweiligen Verschiebungsposition fixierbar. Die Verankerungselemente 16 sind so ausgebildet, dass sie in der Haut 10 verankerbar sind.

In dem dargestellten Ausführungsbeispiel weisen die Verankerungselemente 16 an ihrer Unterseite Haken 18 auf, die in die Haut 10 eingestochen werden, um die Verankerungselemente 16 in der Haut 10 zu verhaken und zu verankern. Andere Ausbildungen der Verankerungselemente sind ebenfalls bekannt und können alternativ zu den Haken 18 verwendet werden. Vorzugsweise können die Verankerungselemente 16 anstelle der Haken auch sicherheitsnadel-förmige Verankerungen aufweisen, wie sie z. B. in der US 4,896,680 gezeigt sind. In dieser Ausführung weisen die Verankerungselemente 16 eine quer zur Längserstreckung des Bandes 14 angeordnete Sicherheitsnadel auf, die in die Haut 10 eingestochen wird. Der Vorteil einer Ausbildung der Verankerungselemente 16 mit Haken 18 besteht darin, dass die Haken 18 einfach in die Haut 10 eingesetzt werden können und mit einer größeren Variationsfreiheit in der Richtung quer zu dem Band 14 angeordnet werden können. Der Vorteil von Verankerungselementen 16 mit einer Sicherheitsnadel besteht vor allem darin, dass sich diese sicherheitsnadel-förmige Verankerung nicht unbeabsichtigt aus der Verankerung in der Haut 10 lösen kann.

Wie die Figuren 1 und 2 zeigen, sitzen auf dem Band 14 zwei erste Verankerungselemente 16.1, die einander gegenüber an den einander gegenüberliegenden Rändern der Wunde 12 in die Haut 10 eingesetzt werden. Die ersten Verankerungselemente 16.1 können auf dem Band 14 gegeneinander geschoben werden, sodass sich ihr gegenseitiger Abstand in Längsrichtung des Bandes 14 verringert und der Zwischenbereich zwischen den ersten Verankerungselementen 16.1, im vorliegenden Fall der Bereich der Wunde 12, verkleinert wird. Die die Ränder der Wunde 12 bildende Haut 10 wird hierdurch in Längsrichtung des Bandes 14 gedehnt, sodass die Wundränder aufeinander zu bewegt werden, um die Wunde 12 zu verschließen. Ist die Haut an den Wundrändern soweit gedehnt, dass sich die einander gegenüberliegenden Wundränder berühren, können die Wundränder zum Verschließen der Wunde 12 miteinander vernäht oder verklammert werden.

Die Dehnung der Haut 10 induziert in dem Bereich, in welchem die Haut 10 gedehnt wird, eine verstärkte Gewebsproliferation, sodass die Dehnung der Haut 10 zu einer Vergrößerung der zur Verfügung stehenden Hautfläche führt. Die Gewebsproliferation ist umso größer, je größer die von den Verankerungselementen 16 auf die Haut ausgeübte Zugkraft ist. Bei herkömmlichen Vorrichtungen, die nur jeweils erste Verankerungselemente 16.1 aufweisen, die in der Haut 10 der Wundränder verankert werden, ist die auf die Haut einwirkende Zugkraft begrenzt. Die Verankerungselemente 16, z. B. die Haken 18, üben entsprechend der eingeleiteten Zugkraft auf ihrer in Zugrichtung weisenden Vorderseite einen Druck auf das Gewebe aus. Überschreitet dieser Druck die sog. Ischämie-Grenze, so verhindert dieser Druck eine Durchblutung des Gewebes. Dieser Ischämie-Zustand kann nur eine zeitlich begrenzte Dauer bestehen, da anderenfalls das Gewebe nekrotisch wird. Bei den bekannten Vorrichtungen muss daher die Zugkraft unter der Ischämie-Grenze gehalten werden, was zu einer geringeren Hautdehnung und Gewebsproliferation führt, oder die Zugkraft muss in regelmäßigen Zeitabständen für eine Erholungsphase zur Durchblutung des Gewebes unterbrochen werden, was ebenfalls die Effektivität der Hautdehnung verringert. Schließlich ist die Reichweite der in die Haut eingeleiteten Zugkräfte auf wenige Zentimeter begrenzt. Die über die Verankerungselemente 16 in die Haut 10 eingeleitete Zugkraft nimmt exponentiell mit dem Abstand von dem Verankerungselement 16 ab und ist z. B. bei der menschlichen Haut nach etwa 5 bis 6 cm auf 0 abgefallen. Dies bedeutet, dass nur ein entsprechend begrenztes Hautareal durch die eingeleitete Zugkraft mobilisiert und zur Gewebsproliferation angeregt wird.

Zur Beseitigung bzw. Verringerung dieser Probleme sind erfindungsgemäß zusätzlich zu dem Paar der ersten Verankerungselemente 16.1 wenigstens ein zweites Verankerungselement 16.2 vorgesehen. In dem Ausführungsbeispiel der Figuren 1 und 2 sind jeweils jedem der ersten Verankerungselemente 16.1 ein zweites Verankerungselement 16.2 zugeordnet. Die zweiten Verankerungselemente 16.2 sind vorzugsweise gleich ausgebildet wie die ersten Verankerungselemente 16.1. Die zweiten Verankerungselemente 16.2 können daher ebenfalls auf dem Band 14 verschoben und in ihrer jeweiligen Verschiebungsposition arretiert werden.

In Fig. 1 ist bspw. ein erstes Verankerungselement 16.1 in die Haut 10 des rechten Randes der Wunde 12 eingesetzt. Auf der von der Wunde 12 abgewandten Seite des ersten Verankerungselementes 16.1 ist ein zweites Verankerungselement 16.2 auf dem Band 14 angeordnet. Dieses zweite Verankerungselement 16.2 wird von dem ersten Verankerungselement 16.1 in Längsrichtung des Bandes 14 beabstandet in der Haut 10 verankert. Entsprechend der Reichweite der Zugkräfte in der Haut 10 kann dieser Abstand je nach Anwendungsfall 1 bis 20 cm, für die menschliche Haut im allgemeinen etwa 1,5 bis 3 cm betragen. Das zweite Verankerungselement 16.2 übt auf die Haut 10 eine Zugkraft aus, die durch die Verschiebung des zweiten Verankerungselementes 16.2 auf dem Band 14 gegen die Wunde 12 hin, d. h. in Fig. 1 nach links, erzeugt wird. Damit wird ein durch die Reichweite der Zugkraft bestimmtes zusätzliches Hautareal rechts von dem zweiten Verankerungselement 16 mobilisiert und zur Gewebsproliferation angeregt. Weiter bewirkt das zweite Verankerungselement 16.2 eine Vordehnung der Haut 10 für das am Wundrand angeordnete erste Verankerungselement 16.1. Auf den Hautbereich zwischen dem ersten Verankerungselement 16.1 und dem zweiten Verankerungselement 16.2 wirkt somit eine Zugkraft, die durch eine Verschiebung des ersten Verankerungselementes 16.1 gegenüber dem zweiten Verankerungselement 16.2 bestimmt werden kann.

Eine entsprechende Anordnung eines ersten Verankerungselementes 16.1 und eines zweiten Verankerungselementes 16.2 ist in Fig. 1 auf der linken Seite der Wunde 12 gezeigt. Es ist ohne Weiteres ersichtlich, dass zweite Verankerungselemente 16.2 auch nur auf einer Seite der Wunde 12 vorgesehen sein können, sodass auf einer Wundseite nur ein einziges erstes Verankerungselement 16.1 vorgesehen ist. Weiter ist es ohne Weiteres ersichtlich, dass auch mehr als ein zweites Verankerungselement 16.2 auf einer Seite hinter dem ersten Verankerungselement 16.1 angeordnet sein kann. Sind zwei oder mehr zweite Verankerungselemente 16.2 hintereinander angeordnet, so sind diese unabhängig voneinander in Längsrichtung des Bandes 10 verstellbar und arretierbar und ihre Funktion addiert sich in der zuvor beschriebenen Weise.

Die Vorrichtung der Figuren 1 und 2 bietet folgende Anwendungsmöglichkeiten und Vorteile:

Durch die hintereinander angeordneten Verankerungselemente 16.1 und 16.2 können in Richtung der Längserstreckung des Bandes 14, d. h. in Dehnungsrichtung, größere Hautareale gedehnt und für die Gewebsproliferation aktiviert werden. Da eine größere Gewebsfläche mobilisiert wird, ergibt sich bei gleicher Gewebsproliferation pro Fläche ein größerer Hautgewinn für den Verschluss der Wunde 12.

Die zweiten Verankerungselemente 16.2 erzeugen eine Vordehnung der Haut für die in Zugrichtung folgenden weiteren zweiten Verankerungselemente 16.2 bzw. die ersten Verankerungselemente 16.1. Die durch die einzelnen Verankerungselemente 16.1, 16.2 in die Haut jeweils punktuell eingeleitete Zugkraft kann daher geringer gewählt werden. Auch bei einer geringeren Zugkraft der einzelnen Verankerungselemente kann eine Hautdehnung und ein Hautgewinn erzielt werden, der mit herkömmlichen Mitteln nur mit einer wesentlich höheren Zugkraft erzielt werden kann. Auch wenn die mit den einzelnen Verankerungselementen eingeleitete Kraft unterhalb der Ischämie-Grenze liegt, kann eine effektive Hautgewinnung erreicht werden.

Bei einer weiteren Verwendungsweise der erfindungsgemäßen Vorrichtung können die über das erste Verankerungselement 16.1 und die über die einen oder mehreren zweiten Verankerungselemente 16.2 in die Haut 10 eingeleiteten Zugkräfte zeitlich variiert werden. Bspw. wird in einer ersten Zeitperiode über das erste Verankerungselement 16.1 eine hohe Zugkraft in die Haut 10 eingeleitet, die auf der Druckseite des Verankerungselementes 16.1 die Ischämie-Grenze überschreitet. Das zweite Verankerungselement 16.2 übt hierbei nur eine geringe Zugkraft auf die Haut 10 aus oder ist vollständig entlastet. In einer anschließenden Zeitperiode vertauschen die ersten und zweiten Verankerungselemente ihre Rolle. Das zweite Verankerungselement 16.2 wird so eingestellt, dass es eine sehr hohe Zugkraft in die Haut 10 einleitet, die auf der Druckseite des zweiten Verankerungselementes 16.2 die Ischämie-Grenze überschreiten kann. In dieser Zeitperiode wird die vom ersten Verankerungselement 16.1 eingeleitete Zugkraft reduziert oder vollständig weggenommen. Diese Perioden wiederholen sich dann zyklisch. Auf diese Weise wird jeweils über eines der Verankerungselemente 16.1 bzw. 16.2 eine hohe Zugkraft in die Haut 10 eingeleitet, die zu einer starken Gewebsproliferation führt. Das jeweils andere Verankerungselement 16.1 bzw. 16.2 ist dabei entlastet oder zumindest soweit entlastet, dass das Gewebe auch auf der Druckseite des Verankerungselementes wieder gut und vollständig durchblutet wird und sich regenerieren kann. Insgesamt kann somit während der gesamten Behandlungsdauer die Haut 10 des Wundrandes ununterbrochen mit einer sehr hohen Zugkraft gedehnt werden, wobei die Angriffspunkte dieser Zugkraft zyklisch wechseln, sodass sich das Gewebe stets wieder ausreichend regenerieren kann. Da die hohe Zugkraft ständig mit wechselnden Angriffspunkten auf die Haut 10 einwirkt, wird eine sehr effektive Dehnung und Gewebsproliferation bewirkt.

In Fig. 2 ist eine Ausführung gezeigt, bei welcher jedes Verankerungselement 16.1, 16.2 zwei Haken 18 quer zur Längsrichtung des Bandes 14 nebeneinander aufweist. Eine solche Ausführung ermöglicht die Krafteinleitung in einem relativ schmalen Abschnitt des Wundrandes. Bei einer größeren Wunde 12 kann der Wundrand durch mehrere nebeneinander angeordnete Vorrichtungen individuell gedehnt werden.

Fig. 3 zeigt eine Abwandlung, bei welcher die Verankerungselemente 16.1, 16.2 eine größere Abmessung quer zur Längserstreckung des Bandes 14 aufweisen und somit auch mehrere Haken 18 aufweisen können. Diese Ausführung eignet sich dazu, eine größere Breite des Wundrandes zu dehnen.

Fig. 4 zeigt eine weitere Abwandlung, bei welcher zwei in Dehnungsrichtung parallel zueinander verlaufende Bänder 14 vorgesehen sind, auf welchen die jeweiligen Verankerungselemente 16.1, 16.2 verschiebbar und fixierbar angeordnet sind. Diese Ausführung gibt eine höhere Stabilität bei breiten Verankerungselementen 16.1, 16.2. Außerdem kann das Verankerungselement 16.1, 16.2 mit seinen beiden Enden auf den zwei Bändern 14 unterschiedlich verstellt werden. Damit können die Verankerungselemente 16.1, 16.2 auch gegen die Richtung der Bänder 14 und damit gegen die Dehnungsrichtung schräg gestellt werden, sodass sie dem Verlauf des Wundrandes angepasst werden können.

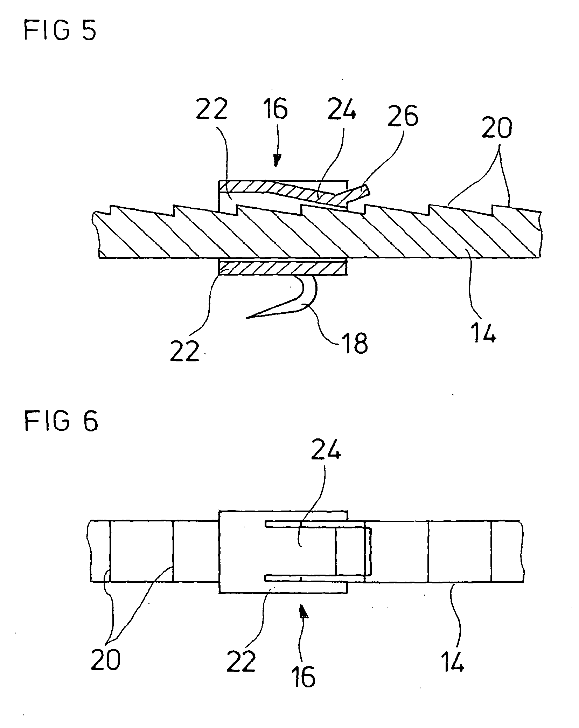

In den Figuren 5 und 6 ist ein Ausführungsbeispiel für ein Verankerungselement 16 und dessen Verschiebbarkeit und Arretierbarkeit auf dem Band 14 gezeigt. Das flexible Band 14 weist an seiner Oberseite Querrippen 20 auf, die bspw. ein Sägezahnprofil bilden können. Das Verankerungselement 16 weist bspw. ein aus Kunststoff gefertigtes, das Band 14 umschließendes kastenförmiges Gehäuse 22 auf. An der Unterseite des Gehäuses 22 sind die Haken 18 eingespritzt. Das Gehäuse 22 ist mit seiner Unterseite und seinen Seitenwänden an dem Band 14 geführt. Die Oberseite des Gehäuses 22 ist mit einer elastisch gegen die Oberseite des Bandes 14 vorgespannten Rastzunge 24 ausgebildet. Die Rastzunge 24 liegt mit einer Rastnase an der Oberseite des Bandes 14 an. Wie aus Fig. 5 ersichtlich ist, ermöglicht die Ausbildung der sägezahn-förmigen Querrippen 20 und der Rastzunge 24 eine Verschiebung des Verankerungselementes auf dem Band 14 nach links. Hierbei gleitet die Rastzunge 24 über die Querrippen 20. Gegen eine Bewegung des Verankerungselementes 16 auf dem Band 14 in Fig. 5 nach rechts ist das Verankerungselement 16 jedoch verriegelt, indem die Rastzunge 24 mit ihrer Rastnase in der Verzahnung der Querrippen 20 einrastet. Eine Handhabungsspitze 26 am freien Ende der Rastzunge 24 ermöglicht es, die Rastzunge aus der Verrastung der Querrippen 20 gegen ihre elastische Kraft herauszuheben, sodass das Verankerungselement 16 aus seiner Arretierposition gelöst werden kann und auf dem Band 14 auch in Fig. 5 nach rechts verschoben werden kann.

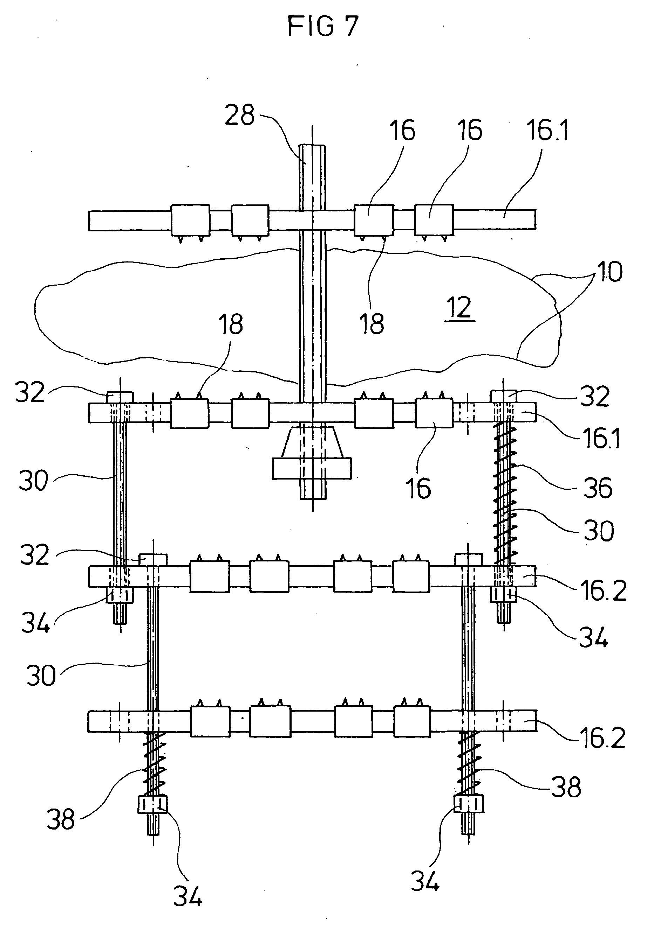

Fig. 7 zeigt eine andere Ausführungsform der Vorrichtung. Soweit diese mit der zuvor beschriebenen Ausführung übereinstimmt, sind dieselben Bezugszeichen verwendet und auf die vorhergehende Beschreibung wird Bezug genommen.

In dem Ausführungsbeispiel der Fig. 7 weist die Führungseinrichtung eine Gewindespindel 28 auf, auf welcher die ersten Verankerungselemente 16.1 gegeneinander verstellbar sind. Die Führung der ersten Verankerungselemente 16.1, die Ausbildung der Gewindespindel 28 und die Verstellbarkeit können bspw. in der aus der DE 44 44 130 A1 bekannten Weise ausgebildet sein. Andere Ausführungen liegen im Bereich des fachmännischen Handelns.

Wie in Fig. 7 nur für eine Seite der Vorrichtung gezeigt ist, kann an dem ersten Verankerungselement 16.1 ein zweites Verankerungselement 16.2 angeschlossen sein. Zur Verbindung und Führung des zweiten Verankerungselementes 16.2 an dem ersten Verankerungselement 16.1 dienen an den äußeren Enden der Verankerungselemente 16.1 und 16.2 angeordnete Gewindestäbe 30. Die Gewindestäbe 30 durchsetzen frei durchgehende Bohrungen der Verankerungselemente 16.1 und 16.2. An dem Verankerungselement 16.1 werden die Gewindestäbe 30 durch einen Kopf 32 gehalten. Auf das entgegengesetzte, durch das zweite Verankerungselement 16.2 geführte Ende des Gewindestabes 30 ist eine Gewindemutter 34 aufgeschraubt. Durch Verstellen der Gewindemutter 34 auf dem Gewindestab 30 kann der Abstand zwischen dem ersten Verankerungselement 16.1 und dem zweiten Verankerungselement 16.2 eingestellt und fixiert werden. In der Dehnungsrichtung, d. h. in der Richtung der Gewindestäbe 30, werden die Verankerungselemente 16.1 und 16.2 durch die elastische Zugspannung der Haut in ihrem durch die Gewindemutter 34 begrenzten Abstand gehalten. Ggf. können die Verankerungselemente 16.1 und 16.2 noch durch eine zusätzlich auf dem Gewindestab 30 sitzende Schraubendruckfeder 36 an dem Kopf 32 und der Gewindemutter 34 anliegend gehalten werden. Durch Verstellen der Gewindemuttern 34 auf den Gewindestäben 30 kann das zweite Verankerungselement 16.2 gegen das erste Verankerungselement 16.1 gezogen werden, um die durch das zweite Verankerungselement 16.2 in die Haut 10 eingeleitete Zugkraft einzustellen. Das erste Verankerungselement 16.1 wird dabei über die Gewindespindel 28 und die Verankerungselemente auf der anderen Seite der Wunde 12 abgestützt.

An das zweite Verankerungselement 16.2 können sich in entsprechender Weise weitere zweite Verankerungselemente 16.2 anschließen, wie dies in Fig. 7 für ein weiteres Verankerungselement 16.2 gezeigt ist. Die Verbindung und die Verstellbarkeit zwischen den aufeinanderfolgenden zweiten Verankerungselementen 16.2 entspricht der Verbindung und Verstellbarkeit zwischen dem ersten Verankerungselement 16.1 und dem zweiten Verankerungselement 16.2.

Bei der Verbindung und Führung der zwei aufeinanderfolgenden zweiten Verankerungselemente 16.2 ist eine weitere Variante dargestellt. Zwischen die Gewindemutter 34 und das Verankerungselement 16.2 ist eine Schraubendruckfeder 38 eingesetzt. Die Schraubendruckfeder 38 drückt das entsprechende Verankerungselement 16.2 in der Zugrichtung der Vorrichtung. Die Schraubendruckfeder 38 bestimmt somit die durch das Verankerungselement 16.2 auf die Haut 10 ausgeübte Zugkraft. Durch die Gewindemutter 34 kann die Schraubendruckfeder 38 vorgespannt werden, um diese eingeleitete Zugkraft einzustellen. Die Schraubendruckfeder 38 hält diese über das Verankerungselement 16.2 in die Haut 10 eingeleitete Kraft auch über einen gewissen Bewegungsweg des Verankerungselementes 16.2 aufrecht. Auch wenn sich die Haut 10 unter der Wirkung der eingeleiteten Zugkraft dehnt und das Verankerungselement 16.2 auf den Gewindestäben 30 wandert, wird die eingeleitete Zugkraft durch die Schraubendruckfeder 38 aufrecht erhalten. Ein Nachstellen der Gewindemuttern 34 zum Einstellen der einwirkenden Zugkraft wird daher erst in größeren Zeitabständen notwendig.

Die Ausführung der Fig. 7 zeigt deutlich, wie durch das jeweilige Verstellen der ersten Verankerungselemente 16.1 auf der Gewindespindel 28 und das gegenseitige Verstellen der Verankerungselemente 16.1 und 16.2 über die Gewindestäbe 30 und die Gewindemuttern 34 auch ein Wechseln zwischen Zugphase und Regenerationsphase durch gegenseitiges Verstellen möglich ist.

Ist die Vorrichtung an beiden Seiten der Wunde 12 im Wesentlichen symmetrisch ausgebildet, wie dies die zuvor beschriebenen Ausführungen zeigen, so wird im Wesentlichen auf die Haut 10 an beiden Wundrändern eine einstellbare Zugkraft ausgeübt. Die Haut 10 wird dementsprechend an beiden Wundrändern gedehnt.

Es gibt jedoch auch Anwendungsfälle, bei denen die Haut möglichst nur an einer Seite der Wunde 12 gedehnt werden sollte, während an dem anderen Wundrand eine Hautdehnung unerwünscht ist, z. B. weil unter diesem Hautbereich Druck verursachende Strukturen liegen.

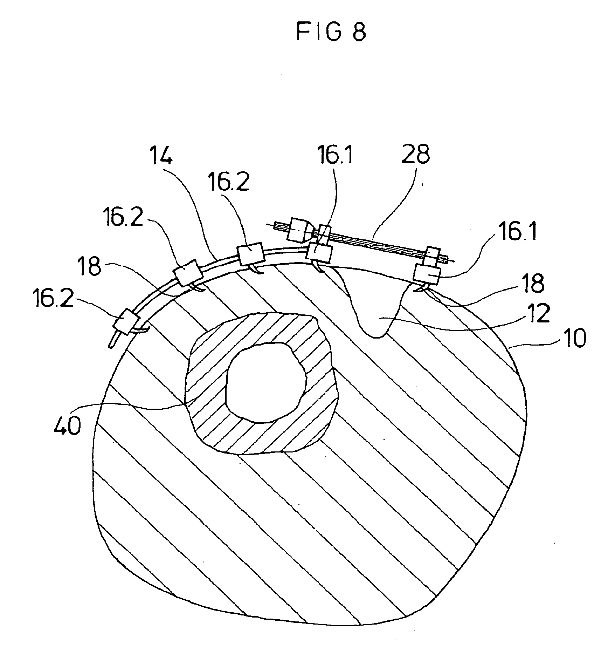

Fig. 8 zeigt einen solchen Anwendungsfall, bei welchem eine Wunde 12 sich angrenzend an die Kante eines Knochens 40 befindet.

In diesem Fall wird die Vorrichtung in dem Wundrand, dessen Haut gedehnt werden soll, mit einem ersten Verankerungselement 16.1 (und ggf. auch mit weiteren zweiten Verankerungselementen 16.2) verankert. Auf der Wundseite, auf welcher die Haut 10 nicht gedehnt werden soll, werden ein erstes Verankerungselement 16.1 und ein oder vorzugsweise mehrere zweite Verankerungselemente 16.2 in der Haut 10 verankert. Die Abstände zwischen dem ersten Verankerungselement 16.1 und den folgenden Verankerungselementen 16.2 werden dabei nicht verstellt, sodass die Haut 10 zwischen dem ersten Verankerungselement 16.1 und den aufeinanderfolgenden Verankerungselementen 16.2 keiner Zugkraft ausgesetzt wird und nicht gedehnt wird. Die Verankerungselemente 16.2 bewirken somit eine möglichst großflächige zugfreie Verankerung für das erste Verankerungselement 16.1. Werden die Verankerungselemente 16.1, die jeweils an den einander gegenüberliegenden Rändern der Wunde 12 verankert sind, mittels der Gewindespindel 28 gegeneinander gezogen, so führt dies asymmetrisch zu einer Dehnung der Haut 10 an dem in Fig. 8 rechten Rand der Wunde 12. Die Gegenkraft zum Abstützen der von dem Verankerungselement 16.1 am rechten Wundrand ausgeübten Zugkraft wird dagegen von dem ersten Verankerungselement 16.1 und den folgenden Verankerungselementen 16.2 auf der in Fig. 8 linken Wundseite auf ein sehr großes Hautareal verteilt, sodass dort praktisch keine Zugkraft durch die einzelnen Verankerungselemente 16.1, 16.2 in die Haut eingeleitet wird.

### Bezugszeichenliste

- 10: Haut
- 12: Wunde
- 14: Band
- 16: Verankerungselemente
- 16.1: erstes Verankerungselement
- 16.2: zweites Verankerungselement
- 18: Haken
- 20: Querrippen
- 22: Gehäuse
- 24: Rastzunge
- 26: Handhabungsspitze
- 28: Gewindespindel
- 30: Gewindestäbe
- 32: Kopf
- 34: Gewindemutter
- 36: Schraubendruckfeder
- 38: Schraubendruckfeder
- 40: Knochen

## Patentansprüche

1. Vorrichtung zum Dehnen der Haut, mit wenigstens einer in der Dehnungsrichtung verlaufenden Führungseinrichtung (14, 28, 30), mit einem Paar von ersten Verankerungselementen (16.1), die in der Haut (10) verankerbar sind und an der Führungseinrichtung (14, 28) geführt in ihrem gegenseitigen Abstand verstellbar angeordnet sind, um einen Zwischenbereich zwischen den ersten Verankerungselementen (16.1) zu verkleinern, und mit wenigstens einem zweiten Verankerungselement (16.2) das außerhalb des Zwischenbereichs an der Führungseinrichtung (14, 30) verstellbar angeordnet ist.

2. Vorrichtung nach Anspruch 1,
**dadurch gekennzeichnet, dass** die Verankerungselemente (16) auf der Führungseinrichtung (14, 28, 30) lösbar arretierbar sind.

3. Vorrichtung nach Anspruch 1 oder 2,
**dadurch gekenntzeichnet**, dass die Führungseinrichtung ein in Dehnungsrichtung verlaufendes flexibles Band (14) aufweist, auf welchem die Verankerungselemente (16) verschiebbar und fixierbar gelagert sind.

4. Vorrichtung nach Anspruch 3,
**dadurch gekennzeichnet, dass** das Band (14) Querrippen (20) aufweist, in welchen die Verankerungselemente (16) einrastbar sind.

5. Vorrichtung nach Anspruch 1 oder 2,
**dadurch gekennzeichnet, dass** die Führungseinrichtung wenigstens eine Gewindespindel (28) aufweist, mittels welcher die Verankerungselemente (16.1) verstellbar sind.

6. Vorrichtung nach einem der vorhergehenden Ansprüche,
**dadurch gekennzeichnet, dass** das erste Verankerungselement (16.1) und ein folgendes zweites Verankerungselement (16.2) und/oder zwei aufeinanderfolgende zweite Verankerungselemente (16.2) mittels Gewindestäben (30) miteinander verbunden sind und mittels auf diesen Gewindestäben (30) angeordneten Gewindemuttern (34) gegeneinander verstellbar sind.

7. Vorrichtung nach einem der vorhergehenden Ansprüche,
**dadurch gekennzeichnet, dass** die Verankerungselemente (16) mittels in die Haut einstechbarer Haken (18) oder Sicherheitsnadeln verankerbar sind.

8. Vorrichtung nach einem der Ansprüche 1 bis 7,
**dadurch gekennzeichnet, dass** die Verankerungselemente (16) manuell verstellbar sind.

9. Vorrichtung nach einem der Ansprüche 1 bis 7,
**dadurch gekennzeichnet, dass** die Verankerungselemente (16) motorisch verstellbar sind.

10. Vorrichtung nach Anspruch 9,
**dadurch gekennzeichnet, dass** Ist-Werte der Hautdehnung gemessen werden und die motorische Verstellung diese Ist-Werte auf vorgebbare SollWerte regelt.

## Claims

1. An apparatus for stretching the skin, with at least one guiding device (14, 28, 30) extending in the stretching direction, with a pair of first anchoring elements (16.1) which are capable of being anchored in the skin (10) and are arranged so as to be adjustable in their mutual distance in a manner guided on the guiding device (14, 28), in order to reduce an intermediate area between the first anchoring elements (16.1), and with at least one second anchoring element (16.2) which is arranged so as to be adjustable on the guiding device (14, 30) outside the intermediate area.

2. An apparatus according to claim 1, **characterized in that** the anchoring elements (16) are capable of being locked in a releasable manner on the guiding device (14, 28, 30).

3. An apparatus according to claim 1 or 2, **characterized in that** the guiding device has a flexible band (14) which extends in the stretching direction and on which the anchoring elements (16) are mounted so as to be capable of being displaced and fixed.

4. An apparatus according to claim 3, **characterized in that** the band (14) has transverse ribs (20) in which the anchoring elements (16) are capable of engaging.

5. An apparatus according to claim 1 or 2, **characterized in that** the guiding device has at least one threaded spindle (28), by means of which the anchoring elements (16.1) are adjustable.

6. An apparatus according to any one of the preceding claims, **characterized in that** the first anchoring element (16.1) and a following second anchoring element (16.2) and/or two following second anchoring elements (16.2) are connected to one another by means of threaded rods (30) and are adjustable with respect to one another by means of threaded nuts (34) arranged on these threaded rods (30).

7. An apparatus according to any one of the preceding claims, **characterized in that** the anchoring elements (16) are capable of being anchored by means of hooks (18) or safety pins capable of piercing the skin.

8. An apparatus according to any one of claims 1 to 7, **characterized in that** the anchoring elements (16) are adjustable manually.

9. An apparatus according to any one of claims 1 to 7, **characterized in that** the anchoring elements (16) are adjustable by a motor.

10. An apparatus according to claim 9, **characterized in that** actual values of the stretching of the skin are measured and the adjustment by a motor regulates these actual values to pre-settable nominal values.

## Revendications

1. Dispositif pour tendre la peau comportant au moins une installation de guidage (14, 28, 30) dirigée dans la direction d'extension,
une paire de premiers éléments d'accrochage (16.1) qui s'accrochent dans la peau (10) et sont guidés par l'installation de guidage (14, 28) en étant montés avec un écartement réciproque réglable, pour réduire une
zone intermédiaire entre les premiers éléments d'accrochage (16.1) et au moins un second élément d'accrochage (16.2) monté réglable en dehors de la zone intermédiaire sur l'installation de guidage (14, 30).

2. Dispositif selon la revendication 1,
**caractérisé en ce que**
les éléments d'accrochage (16) se bloquent de manière amovible sur l'installation de guidage (14, 28, 30).

3. Dispositif selon la revendication 1 ou 2,
**caractérisé en ce que**
l'installation de guidage comporte une bande souple (14) dirigée dans la direction d'allongement et portant les éléments d'accrochage (16) de façon coulissante et de manière à pouvoir être bloqués.

4. Dispositif selon la revendication 3,
**caractérisé en ce que**
la bande (14) comporte des nervures transversales (20) dans lesquelles l'accrochent les éléments d'accrochage (16).

5. Dispositif selon la revendication 1 ou 2,
**caractérisé en ce que**
l'installation de guidage comporte au moins une broche filetée (28) permettant de régler les éléments d'accrochage (16.1).

6. Dispositif selon l'une des revendications précédentes,
**caractérisé en ce que**
le premier élément d'accrochage (16.1) et un second élément d'accrochage (16.2) suivant et/ou deux seconds éléments d'accrochage (16.2) successifs sont reliés par des tiges filetées (30) et peuvent être réglées l'une par rapport à l'autre par des écrous (34) portés par les tiges filetées (30).

7. Dispositif selon l'une des revendications précédentes,
**caractérisé en ce que**
les éléments d'accrochage (16) s'accrochent dans la peau par des crochets (18) ou épingles de sécurité.

8. Dispositif selon l'une des revendications 1 à 7,
**caractérisé en ce que**
les éléments d'accrochage (16) sont à réglage manuel.

9. Dispositif selon l'une des revendications 1 à 7,
**caractérisé en ce que**
les éléments d'accrochage (16) sont à réglage motorisé.

10. Dispositif selon la revendication 9,
**caractérisé en ce qu'**
on mesure la valeur réelle de l'allongement de la peau et on règle le réglage motorisé de cette valeur réelle sur une valeur de consigne.
